# EUROPEAN PATENT SPECIFICATION

(11) **EP 0 428 125 B1**
(45) Date of publication and mention of the grant of the patent: **22.06.1994**
(21) Application number: 90121672.1
(22) Date of filing: 13.11.1990
(51) Int. Cl.: A61B 5/103

(54) **Device for performing diagnostic tests on the sensitivity to thermal stimuli**
Diagnostik-Gerät zur Bestimmung der Temperatur-Empfindlichkeit
Dispositif diagnostique destiné à la détermination de la sensibilité thermique

(30) Priority: 16.11.1989 CH 4124/89
(43) Date of publication of application: 22.05.1991
(73) Proprietor: FIDIA S.p.A., I-35031 Abano Terme (Padova) (IT)
(72) Inventor: Assal, Jean-Philippe, CH-1231 Conches/GE (CH); Liniger, Carol, CH-1266 Duillier/VD (CH)
(74) Representative: Gervasi, Gemma, Dr.

(56) References cited:
- EP-A- 0 364 158
- FR-A- 2 585 232
- US-A- 3 274 995
- US-A- 4 787 397

## Description

It is an object of the present invention to provide a device for performing diagnostic tests on the sensitivity of patients to thermal stimuli, which device is portable, handy, and suitable for testing in a very rapid succession at four different temperatures of pre-established level. It allows therefore to perform a complete series of significant evaluations apt to supply a reliable information on the patient's sensitivity in a short time, of the order of minutes.

The device comprises an hermetically sealed box generally having a shape of a parallelepiped, of small size (of the order of a few centimeters) provided with a handle for holding it and with four sensing elements, of cylindrical shape, protruding from said box and each terminating in a metal cap which is put in contact with the patient's epidermis, to supply the thermal stimulus which is employed for evaluating the sensitivity.

The cap's surface is of the order of 5 cm².

The external structure of the sealed box and the sides of the cylindrical elements are made out of a thermally and electrically insulating material.

Each sensing element has in its interior an independent device for electrical heating and for controlling the temperature. The sealed box has a pilot light which indicates its functioning and steady state.

Three elements are pre-set for operating at three fixed standard temperatures.

It was found particularly useful to set those temperatures at 25°, 42° and 50°C respectively. The fourth element may operate at a temperature selected within a 10°C range, in particular between 25° and 35°C, the temperature values corresponding to integers.

The device further comprises a box for the electrical controls for the supply of low voltage current to the above said sensing elements and for regulating the temperature (between 25° and 35°C) of the 4th element, as well as the electric connecting cable.

The temperature variations induced in the sensing elements by the contact with the patient's epidermis are rapidly nullified by the heating and control device, reinstating the standard temperature in a very short time, of the order of a few seconds (maximum 10˝ for the sensing element at 50°C). This allows to make the evaluations at a certain temperature within short intervals. As with this device there is always at disposal four sensing elements constantly in a steady state at four different temperatures, tests may be carried out at different temperatures within short times (of the order of minutes).

The attached figures show a purely illustrative graphic representation of the device according to the invention.

Fig. 1 shows the hermetically sealed box 1, with handle 6, four sensing elements 2, 3, 4 and 5 and the connecting cable 7.

Fig. 2 shows the box for the electric controls, with cable 12 connecting to the 220 V main line, pilot light 10 and knob 9 for selecting the temperature between 25 and 35°C for the fourth sensing element.

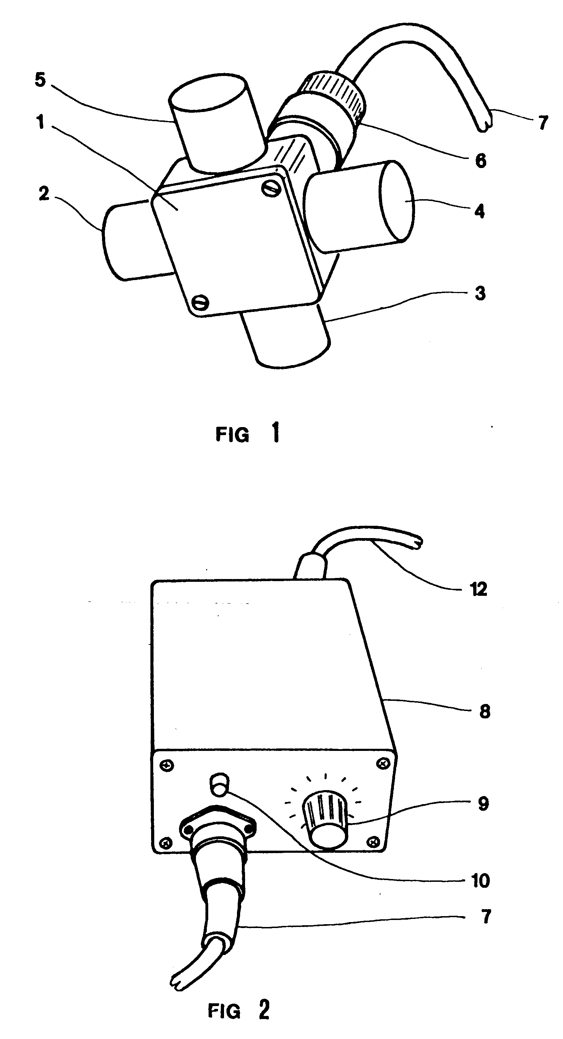

## Claims

1. Portable device for performing diagnostic tests on the sensitivity of patients to thermal stimuli, comprising a hermetically sealed box (1) having a shape of a parallelepiped, provided with a handle for holding it and with four cylindrical sensing elements (2, 3, 4, 5) protruding from said box and each terminating with a metal cap which is to be put in contact with the patient's epidermis, the external structure of said box and the sides of the cylindrical sensing elements being made out of a thermally and electrically insulating material, said sensing elements being each independently provided with electrically heating means and temperature control means; three of the sensing elements are set to operate at three different fixed standard temperatures, while the fourth has a preestablished operating temperature selected within a 10°C range.

2. Device according to claim 1, wherein the three sensing elements set at standard fixed temperatures are kept at 25°, 42° and 50°C respectively, while the fourth is kept at a temperature selected in the 25° to 35°C range.

## Patentansprüche

1. Tragbare Vorrichtung zum Durchführen von Diagnose-Tests der Empfindlichkeit von Patienten auf Wärmereize mit einem hermetisch abgedichteten Gehäuse (1), das die Form eines Quaders hat und zu seiner Handhabung mit einem Handgriff versehen ist sowie mit vier zylindrischen Fühlelementen (2, 3, 4, 5), die sich vom Gehäuse wegerstrecken und die jeweils in einer Metallkappe enden, die mit der Haut des Patienten in Berührung zu bringen sind, wobei der äußere Aufbau des Gehäuses und der Seiten der zylindrischen Fühlelemente aus einem thermisch und elektrischen Isolierungsmaterial bestehen, wobei die Fühlelemente aus einem thermisch elektrischen Isolierungsmaterial bestehen, wobei die Fühlelemente jeweils unabhängig voneinander mit elektrischen Heiz- und Temperatursteuervorrichtungen versehen sind und wobei drei der Fühlelemente so eingestellt sind, daß sie bei drei unterschiedlichen, festliegenden Normtemperaturen arbeiten, während das vierte Fühlelement eine voreingestellte Betriebstemperatur hat, die innerhalb eines 10°C-Bereiches ausgewählt worden ist.

2. Vorrichtung nach Anspruch 1, wobei die drei Fühlelemente, die auf festliegende Normtemperaturen eingestellt sind, auf 25°, 42° und 50°C gehalten werden, während das vierte Fühlelemente auf einer Temperatur gehalten wird, die im Bereich zwischen 25° und 35°C ausgewählt ist.

## Revendications

1. Dispositif portable pour la réalisation de tests diagnostiques sur la sensibilité des patients à des stimuli thermiques, comprenant une boîte hermétiquement fermée (1), ayant la forme d'un parallélépipède, munie d'une poignée pour la tenir et de quatre capteurs cylindriques (2, 3, 4, 5) faisant saillie sur ladite boîte et se terminant chacun par un capuchon métallique à mettre en contact avec l'épiderme du patient, la structure externe de la boîte et les parois des capteurs cylindriques étant réalisées en un matériau isolant thermique et électrique, chacun des capteurs étant muni indépendamment de moyens de chauffage électrique et de moyens de contrôle de la température ; trois des capteurs sont préréglés pour fonctionner à trois températures standard différentes fixées, alors que le quatrième capteur a une température de fonctionnement prédéterminée choisie dans un intervalle de 10°C.

2. Dispositif selon la revendication 1, dans lequel les trois capteurs réglés à des températures standard fixées sont maintenus respectivement à 25°C, 42°C et 50°C, alors que le quatrième est maintenu à une température choisie dans l'intervalle de 25°C à 35°C.
